# EUROPEAN PATENT SPECIFICATION

(11) **EP 1 569 564 B1**
(45) Date of publication and mention of the grant of the patent: **07.03.2007**
(21) Application number: 03761503.6
(22) Date of filing: 25.06.2003
(51) Int. Cl.: A61B 17/06

(54) **ATRAUMATIC TWO-TIPPED SURGICAL NEEDLE**
ATRAUMATISCHE CHIRURGISCHE NADEL MIT ZWEI SPITZEN
AIGUILLE CHIRURGICALE A DEUX POINTES ATRAUMATIQUE

(30) Priority: 26.06.2002 IT GE20020056
(43) Date of publication of application: 07.09.2005
(73) Proprietor: Capurro, Sergio, 16147 Genoa (IT)
(72) Inventor: Capurro, Sergio, 16147 Genoa (IT)
(74) Representative: Forattini, Amelia
(86) International application number: PCT/EP2003/006685
(87) International publication number: WO 2004/002326

(56) References cited:
- EP-A- 0 205 811
- DE-A- 19 706 086
- DE-U- 29 806 617
- US-A- 5 908 428

## Description

The present invention concerns an atraumatic two-tipped needle for use in surgery.

Atraumatic needles for surgical suturing are already in use. These do not cause trauma as they have no eye, the thread emerging directly from one extremity of the needle, which consists of a solid metallic structure. However, such needles cannot be used to create an invisible suspension of the integument, in that it is practically impossible to re-insert the needle into the same tunnel from which it has emerged; therefore, retraction of the skin due to the tension of the thread will always remain visible.

To overcome this problem, a two-tipped surgical needle was created for use in mini-invasive surgery for suturing subcutaneous wounds. This needle is described in the patent for industrial model N° 194882, registered in the name of the same inventor. That model enables the surgeon to carry out internal suturing or to place a thread in the subcutaneous tissues, according to the desired trajectory, by means of simple external manoeuvres, without leaving any part of the thread visible externally and without needing to make any incisions in the skin. To achieve this, the surgeon inserts the tip of the needle into the tissue and makes it emerge in the direction in which the thread is to pass, but without extracting the needle completely. The other tip of the needle, which remains in the tissue, is then pushed in the desired direction in such a way as to position the thread below the cutaneous layer without leaving visible signs on the surface.

The main disadvantage of that model, however, is that the eye of the needle is larger than the mean diameter of the needle itself. This means not only that penetration of the skin becomes more difficult, but also that considerable force is required to make the needle re-emerge partially; indeed, the force exerted may cause the needle to emerge completely, thereby nullifying any benefit and forcing the surgeon to repeat the procedure from the beginning.

A second disadvantage is that the eye of the needle constitutes a point of mechanical weakness. When the needle is forced to change direction inside the subcutaneous tissue, it is subjected to considerable stress, which might cause it to snap, leading to surgical and legal complications.

A further drawback lies in the cost of producing such needles. Given that this is a specialist tool which has a very small market niche, the cost of manufacturing a two-tipped needle with a centrally-positioned eye industrially is not justified.

All of the above disadvantages have been overcome by the invention of an atraumatic two-tipped surgical needle without an eye.

The atraumatic two-tipped needle in question consists of a tubular metal element, the ends of which are bevelled in one or more oblique planes, rather like the point of a hypodermic needle; the central portion of the shaft is pierced by a small hole, through which the surgical thread emerges, the thread being anchored inside the needle itself. Only one wall of the tubular shaft needs to be pierced, though both may be pierced if this is simpler.

The atraumatic two-tipped surgical needle described here can be used in numerous procedures, such as suspension and tensioning of the integument, elimination of wrinkles from the face and neck (aesthetic lifting), correction of facial nerve paralysis, and modification of the shape of subcutaneous tissues.

Moreover, the needle may also find application in the neurological field through the use of special conducting threads designed to carry electrical impulses from a generator implanted in the body to the most distant tissues.

The first of the many advantages that the present invention offers, in comparison with the traditional two-tipped needle, is that the absence of the eye and the uniform diameter of the tubular metal shaft cause less damage to the tissue that is penetrated.

Secondly, the bevelled tip, like that of a hypodermic needle, facilitates penetration and causes less damage to the tissues.

A further, and particularly important, advantage lies in the fact that a tubular metal structure, whatever size it may be, is more resistant to bending stress than a solid metal shaft.

All of the above advantages, and several others, will emerge from the description of the following figures, that are enclosed to illustrate and not limit, in which:
Fig. 1 shows a sketch of the atraumatic two-tipped needle which is the subject of the present invention.
Fig. 2 shows a longitudinal section illustrating how the surgical thread may be anchored inside the atraumatic two-tipped needle.
Fig. 3 shows a longitudinal section illustrating another method of anchoring the surgical thread inside the atraumatic two-tipped needle.
Fig. 4 illustrates, in a longitudinal section, a variant of the example shown in figure 3

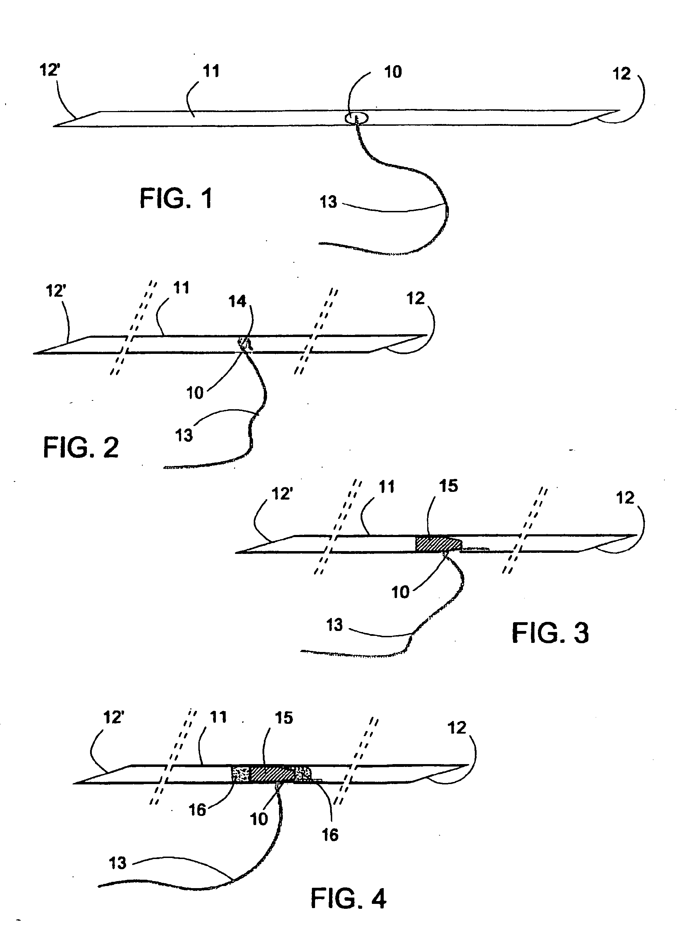

Figure 1 depicts an example of the atraumatic two-tipped needle (11), in accordance with the present invention, in which the hole (10) is located centrally between the two tips (12 and 12'). The hole (10) may be created by using various techniques, such as laser boring, milling, chemical or electrochemical processes, or a combination of techniques. The rim of the hole (10) is ground perfectly smooth so as to eliminate any sharp edges, burrs or imperceptible irregularities.

To anchor the surgical thread (13) inside the needle (11), as in the present invention, various techniques may be used.

An example which is not illustrated here involves inserting one end of the surgical thread (13) into the hole (10) of the needle (11) and fixing it by means of manual or automatic pinching in proximity to the hole (10).

Figure 2 shows a longitudinal section of the needle (11) to exemplify another method of anchoring the surgical thread (13). In this case, the thread (13) is fixed inside the atraumatic two-tipped needle (11) by means of a knot (14), which prevents the thread (13) from slipping out of the hole (10) of the atraumatic two-tipped needle (11). For instance, one end of the surgical thread (13) may be inserted into the hole (10) in the needle (11) and pushed out through one end (12 or 12'); a knot (14) can be tied in the thread (13), which is then pulled back towards the hole (10). Alternatively, a knot (14) can first be tied in the surgical thread (13), which is then inserted through one end of the needle (11) and pulled out through the hole (10).

Figure 3 shows another example of how the surgical thread (13) can be anchored inside the atraumatic two-tipped needle (11). One end of the thread (13) is inserted into the hole (10) and is kept in place by means of a scotch (15), which is pushed down inside the needle (11) from one end (12'). The scotch (15) may consist of a solid bar or a portion of tube, the diameter of which will depend on the diameter of the needle (11) and which may be made of metal or plastic, for example. Once the scotch (15) is in position, it can be held fast inside the needle (11) by means of slight pinching in the vicinity of the hole (10) or by the application of a glue (16), as in Figure 4. The scotch (15) also reinforces the central part of the atraumatic two-tipped needle (11), where the greatest stress is applied as the needle changes direction inside the subcutaneous tissue.

The surgical thread (13) can also be fixed to the needle (11) simply by means of glue or any suitable solidifying material.

The surgical thread (13) can be fixed inside the atraumatic two-tipped needle (11) in various other ways. For example, after emerging from one end (12) of the needle (11), the end of the surgical thread (13) can be inserted into a hole made in one end of a scotch and secured by pinching; the scotch is then inserted into the needle (11).

Alternatively, the surgical thread (13) can be fixed between the coils of a tiny spring, which is then inserted into the atraumatic two-tipped needle (11).

Two or more methods of anchoring the surgical thread (13) can be used together.

The present invention includes all variations in detail and all modifications that may prove obvious to a technician in this field, and which do not lie outside the scope of the present invention, and which are understood to be included within the area of the following claims.

## Claims

1. Surgical needle (11) the central portion of which is equipped with a hole (10), through which emerges a surgical thread (13) that is anchored inside the needle (11) **characterised by** the fact that it is atraumatic, that it has two tips (12, 12') and that it consists of a tubular metal shaft. portion of which is equipped with a hole (10), through which emerges a surgical thread (13) that is anchored inside the needle (11)

2. Needle (11) as claimed in claim 1, **characterised by** the fact that its tips (12, 12') are bevelled in one or more oblique planes and that the hole (10) through which the thread passes involves only one wall of the hollow shaft.

3. Needle (11) as claimed in claim 1, **characterised by** the fact that one end of the surgical thread (13) is inserted into the hole (10) of the needle (11) and is anchored by means of pinching.

4. Needle (11) as claimed in claim 1, **characterised by** the fact that the surgical thread (13) is equipped with a knot (14).

5. Needle (11) as claimed in claim 1, **characterised by** the fact that the end of the surgical thread (13) is inserted into the hole (10) of the atraumatic two-tipped needle (11), is made to emerge from one end (12), is equipped with a knot (14) and is then drawn back inside the shaft towards the hole (10).

6. Needle (11) as claimed in claim 1, **characterised by** the fact that the surgical thread (13), equipped in advance with a knot (14), is inserted into one end of the needle (11) and is made to emerge through the hole (10).

7. Needle (11) as claimed in claim 1, **characterised by** the fact that the end of the surgical thread (13) is inserted into the hole (10) and is anchored inside the atraumatic two-tipped needle (11) by means of a scotch (15), which is pushed down inside the needle from one of the two ends (12, 12').

8. Needle (11) as claimed in claims 1 and 5, **characterised by** the fact that the scotch (15) may consist of a solid bar or a portion of tube, made of metal or plastic, the calibre of which is determined by the diameter of the needle (11).

9. Needle (11) as claimed in claim 1, **characterised by** the fact that the scotch (15), when it has been placed in its final position, is anchored inside the needle (11) by pinching, or by means of glue (16) or other suitable fixing material.

10. Needle (11) as claimed in claim 1, **characterised by** the fact that the surgical thread (13) is fixed to the needle (11) by means of a single- or dual-component glue or other solidifying material suited to the purpose.

11. Needle (11) as claimed in claim 1, **characterised by** the fact that the end of the surgical thread (13) inserted into the hole (10) emerges from one end (12, 12') of the needle (11), and is inserted and fixed into a hole in one end of a scotch, which is subsequently inserted into the needle (11).

12. Needle (11) as claimed in claim 1, **characterised by** the fact that the surgical thread (13) is fixed between the coils of a tiny spring, which is then inserted into the atraumatic two-tipped needle (11).

13. Needle (11) as claimed in claim 1, **characterised by** the fact that that the surgical thread (13) is fixed by means of two or more anchoring techniques.

## Patentansprüche

1. Chirurgische Nadel (11), deren Mittelteil mit einer Bohrung (10) ausgerüstet ist, durch welche ein chirurgischer Faden (13) treten kann, der innerhalb der Nadel (11) verankert ist, **dadurch gekennzeichnet, daß** sie atraumatisch ist, zwei Spitzen (12, 12') aufweist und aus einem röhrenförmigen Metallschaft besteht.

2. Nadel (11) gemäß Anspruch 1, **dadurch gekennzeichnet, daß** die Spitzen (12, 12') in einer oder mehreren schiefen Ebenen abgeschrägt sind und daß die Bohrung (10), durch welche der Faden läuft, nur eine Wandung des hohlen Schafts durchdringt.

3. Nadel (11) gemäß Anspruch 1, **dadurch gekennzeichnet, daß** ein Ende des chirurgischen Fadens (13) in die Bohrung (10) der Nadel (11) eingeführt ist und durch Einklemmen befestigt ist.

4. Nadel (11) gemäß Anspruch 1, **dadurch gekennzeichnet, daß** der chirurgische Faden (13) mit einem Knoten (14) ausgerüstet ist. ,

5. Nadel (11) gemäß Anspruch 1, **dadurch gekennzeichnet, daß** das Ende des chirurgischen Fadens (13) in die Bohrung (10) der atraumatischen, zweispitzigen Nadel (11) eingeführt ist, an einem Ende (12) austritt, mit einem Knoten (14) ausgerüstet ist und dann durch die Bohrung (10) in den Schaft zurückgeführt ist.

6. Nadel (11) gemäß Anspruch 1, **dadurch gekennzeichnet, daß** der chirurgische Faden (13), vorher ausgerüstet mit einem Knoten (14), in ein Ende der Nadel (11.) eingeführt wird und durch die Bohrung (10) ausgeführt wird.

7. Nadel (11) gemäß Anspruch 1, **dadurch gekennzeichnet, daß** der chirurgische Faden (13) durch die Bohrung (10) eingeführt ist und innerhalb der atraumatischen zweispitzigen Nadel (11) durch ein Fixiermittel (15) befestigt ist, welches in die Nadel von einem der beiden Enden (12, 12') eingedrückt wird.

8. Nadel (11) gemäß Anspruch 1 und 5, **dadurch gekennzeichnet, daß** das Fixiermittel (15) aus einem festen Holm oder ein Teil einer Röhre, hergestellt aus Metall oder Plastik, besteht und die Größe durch den Durchmesser der Nadel (11) bestimmt wird.

9. Nadel (11) gemäß Anspruch 1, **dadurch gekennzeichnet, daß** das Fixiermittel (15), wenn in die letztendliche Position gebracht, innerhalb der Nadel (11) durch Einklemmen oder durch Klebstoff (16) oder durch andere geeignete Fixierungsmaterialien befestigt ist.

10. Nadel (11) gemäß Anspruch 1, **dadurch gekennzeichnet, daß** der chirurgische Faden (13) mit der Nadel (11) mittels eines Ein- oder Zweikomponentenklebers oder anderer für die Verwendung geeignete verfestigende Materialien befestigt ist.

11. Nadel (11) gemäß Anspruch 1, **dadurch gekennzeichnet, daß** der chirurgische Faden (13), welcher in die Bohrung (10) eingeführt ist, an einem Ende (12, 12') der Nadel (11) austritt und eingeführt und fixiert ist in eine Bohrung in einem Ende eines Fixiermittels, welches anschließend in die Nadel (11) eingeführt wird.

12. Nadel (11) gemäß Anspruch 1, **dadurch gekennzeichnet, daß** der chirurgische Faden (13) zwischen den Spulen einer kleinen Feder befestigt ist, welche dann in die atraumatische zweispitzige Nadel (11) eingeführt wird.

13. Nadel (11) gemäß Anspruch 1, **dadurch gekennzeichnet, daß** der chirurgische Faden (13) mittels zwei oder mehr Befestigungstechniken fixiert ist.

## Revendications

1. Aiguille chirurgicale (11), dont la partie centrale est dotée d'un trou (10), à travers lequel sort un fil chirurgical (13) qui est ancré à l'intérieur de l'aiguille (11), **caractérisée en ce qu'**elle est atraumatique, **en ce qu'**elle a deux pointes (12, 12'), et **en ce qu'**elle se compose d'une tige métallique tubulaire.

2. Aiguille (11) selon la revendication 1, **caractérisée en ce que** ses pointes (12, 12') sont biseautées en un ou plusieurs plans obliques et **en ce que** le trou (10) à travers lequel le fil passe n'implique qu'une seule paroi de la tige creuse.

3. Aiguille (11) selon la revendication 1, **caractérisée en ce qu'**une extrémité du fil chirurgical (13) est insérée dans le trou (10) de l'aiguille (11) et est ancrée au moyen de pincement.

4. Aiguille (11) selon la revendication 1, **caractérisée en ce que** le fil chirurgical (13) est doté d'un noeud (14).

5. Aiguille (11) selon la revendication 1, **caractérisée en ce que** l'extrémité du fil chirurgical (13) est insérée dans le trou (10) de l'aiguille atraumatique (11) à deux pointes, est faite pour sortir par une extrémité (12), est dotée d'un noeud (14) et est ensuite retirée à l'intérieur de la tige vers le trou (10).

6. Aiguille (11) selon la revendication 1, **caractérisée en ce que** le fil chirurgical (13), doté à l'avance d'un noeud (14), est inséré dans une extrémité de l'aiguille (11) et est fait pour sortir par le trou (10).

7. Aiguille (11) selon la revendication 1, **caractérisée en ce que** l'extrémité du fil chirurgical (13) est insérée dans le trou (10) et est ancrée à l'intérieur de l'aiguille atraumatique (11) à deux pointes au moyen d'une cale de blocage (15) qui est abaissée à l'intérieur de l'aiguille à partir de l'une des deux extrémités (12, 12').

8. Aiguille (11) selon les revendications 1 et 5, **caractérisée en ce que** la cale de blocage (15) se compose d'une barre pleine ou d'une partie de tube, réalisée à partir de métal ou de plastique, dont le calibre est déterminé par le diamètre de l'aiguille (11).

9. Aiguille (11) selon la revendication 1, **caractérisée en ce que** la cale de blocage (15), lorsqu'elle a été placée dans sa position définitive, est ancrée à l'intérieur de l'aiguille (11) par pincement, ou au moyen de colle (16) ou d'un autre matériau de fixation approprié.

10. Aiguille (11) selon la revendication 1, **caractérisée en ce que** le fil chirurgical (13) est fixé sur l'aiguille (11) au moyen d'une colle à un ou deux composants ou d'un autre matériau qui se solidifie, approprié pour ce but.

11. Aiguille (11) selon la revendication 1, **caractérisée en ce que** l'extrémité du fil chirurgical (13) insérée dans le trou (10) sort par une extrémité (12, 12') de l'aiguille (11) et est insérée et fixée dans un trou dans une extrémité d'une cale de blocage, qui est ensuite insérée dans l'aiguille (11).

12. Aiguille (11) selon la revendication 1, **caractérisée en ce que** le fil chirurgical (13) est fixé entre les spires d'un ressort minuscule, qui est ensuite introduit dans l'aiguille atraumatique (11) à deux pointes.

13. Aiguille (11) selon la revendication 1, **caractérisée en ce que** le fil chirurgical (13) est fixé au moyen de deux techniques d'ancrage ou plus.
